# EUROPEAN PATENT SPECIFICATION

(11) **EP 2 156 820 B1**
(45) Date of publication and mention of the grant of the patent: **22.08.2012**
(21) Application number: 08764523.0
(22) Date of filing: 22.05.2008
(51) Int. Cl.: A61K 8/64, A23K 1/16, A23K 1/175, A23L 1/30, A23L 1/305, A23L 2/52, A61K 8/19, A61K 8/26, A61K 8/27, A61K 9/08, A61K 33/06, A61K 33/10, A61K 33/24, A61K 33/26, A61K 33/30, A61K 33/32, A61K 33/34, A61K 38/16, A61P 17/00, A61Q 19/02

(54) **SKIN WHITENING AGENT**
HAUTBLEICHUNGSMITTEL
AGENT DE BLANCHIMENT DE LA PEAU

(30) Priority: 25.05.2007 JP 2007138522
(43) Date of publication of application: 24.02.2010
(73) Proprietor: Megmilk Snow Brand Co., Ltd., Sapporo-shi Hokkaido 0650043 (JP)
(72) Inventor: WATANABE, Tatsuya, Fuchu-shi Tokyo 183-0052 (JP); KATO, Ken, Kawagoe-shi Saitama 350-1165 (JP); UENO, Hiroshi, Kawagoe-shi Saitama 350-1165 (JP); HARUTA, Yuko, Kawagoe-shi Saitama 350-1165 (JP); UEDA, Noriko, Kawagoe-shi Saitama 350-1165 (JP); YOSHIOKA, Toshimitsu, Kawagoe-shi Saitama 350-1165 (JP)
(74) Representative: Forstmeyer, Dietmar
(86) International application number: PCT/JP2008/059462
(87) International publication number: WO 2008/146710

(56) References cited:
- EP-A1- 1 040 766
- JP-A- 9 157 296
- JP-A- 04 059 714
- JP-A- 05 320 068
- JP-A- 2000 281 586
- JP-A- 2000 290 193
- JP-A- 2000 290 194
- JP-A- 2004 269 372
- JP-A- 2006 069 995
- US-A- 5 389 611
- US-A- 5 606 086
- US-A1- 2002 076 384

## Description

### Technical Field

The present invention relates to a whitening agent which has an excellent skin whitening effect and is useful for the prevention and treatment of spots, freckles, and the like. Further, the present invention relates to a food, a drink, a feed, a cosmetic, and a drug, each containing the whitening agent.

### Background Art

The color of the skin is mainly determined depending on the types and amounts of coloring components such as melanin, hemoglobin, carotenoid or the like in the epidermis and in the dermis. They are controlled by various external or internal factors. A melanin pigment is synthesized mainly by a melanocyte in the skin and activated by ultraviolet stimulation, hormonal secretion, and stimulating factors released from a surrounding keratinocyte. Although the main function of the melanin pigment is to alleviate skin problems caused by ultraviolet radiation, metabolic disorders such as the excessive synthesis of melanin or the like cause local pigmentation, so-called spots, freckles, and the like, which are a severe cosmetic problem. As methods of improving pigmentation on the skin, there are given a method of inhibiting melanin formation itself by selectively controlling the toxicity to melanocytes and melanogenic pathways in melanocytes, and a method of promoting the reduction or discharge of melanin already formed. In general, L-ascorbic acid having a reduction action and derivatives thereof are widely used as a substance having whitening action, but a sufficiently high whitening effect has not been obtained. Although hydroquinone, albutin, kojic acid, a licorice extract, and a placenta extract which are inhibitors for tyrosinase, a melanin synthesizing enzyme, are used to obtain a whitening effect, those substances have stability and safety problems. Under these circumstances, the development of a whitening agent which is safe, can be ingested or applied on a daily basis, and has an excellent whitening effect is desired.

It is reported that a lactoferrin (s) (hereinafter referred to as LF(s)) is(are) iron-binding proteins separated from secretory fluid such as mammalian milk and the like and have various physiological functions such as an iron absorption promoting action, anti-inflammatory action, lipid peroxide formation inhibiting action, and immune system control action. Various foods, drinks, feeds, and cosmetics have been developed by utilizing the physiological functions of the LFs. Meanwhile, binding properties between the LFs and not only iron but also various metals have been studied, and a method of binding a plurality of metals to the LFs, and, drugs, foods, and drinks for mineral supplementation using the method have been developed.
US 5,389, 611 discloses lactoferrin hydrolyzate for use as an antibacterial agent and as a tyrosinate inhibition agent.

### Disclosure of the Invention

### Problem to be solved by the Invention

It is an object of the present invention to provide a whitening agent which has high safety and an excellent whitening effect for the skin.

### Means for solving the Problems

The inventors of the present invention have conducted intensive studies to search for a material which is safe, can be ingested and applied on a daily basis, and has an excellent whitening effect, and found that a carbonic acid and/or bicarbonic acid-metal-LFs complex, a degradation product of the complex, and a carbonic acid and/or bicarbonic acid-metal-LFs degradation product complex have an excellent whitening effect. Thus, the finding led to the completion of the present invention.

The carbonic acid and/or bicarbonic acid-metal- LFs complex which is the active ingredient of the whitening agent of the present invention can be prepared by a method described in Japanese Patent No. 2884045, Japanese Patent No. 3223958, or the like. For example, the complex can be prepared by adding a solution containing a carbonic acid ion, a bicarbonic acid ion, or a carbonic acid ion and a bicarbonic acid ion and a solution containing LFs to a solution containing one kind or multiple kinds of metals selected from iron, copper, zinc, manganese, cobalt, nickel, and aluminum and mixing them together.

The LFs in the present invention is iron binding proteins generally called "transferrin family", and an LF separated from secretory fluid such as milk of mammalian such as a human, bovine or the like, transferrin separated from the blood, the internal organ, or the like, or ovotransferrin separated from an egg, for example, may be used. A number of methods for preparing those LFs in large amounts have already been known, and LFs prepared by any method may be used. The LFs do not need to be isolated completely and may contain other components. Further, LFs produced from a microorganism, an animal cell, or a transgenic animal by gene manipulation may also be used.

The carbonic acid and/or bicarbonic acid-metal-LFs degradation product complex which is the active ingredient of the whitening agent of the present invention can be prepared by mixing a solution containing a carbonic acid ion and/or a bicarbonic acid ion, a solution containing one kind or multiple kinds of metals, and a solution containing a LFs degradation product. The LFs degradation product is obtained by decomposing LFs with a protein degrading enzyme such as trypsin, pepsine, chymotrypsin or the like, or with an acid or alkali. A degradation product having a molecular weight of about 1,000 Da or more to about 70, 000 Da or less is preferred. Further, the degradation product of the carbonic acid and/or bicarbonic acid-metal-LFs complex can be prepared by decomposing a carbonic acid and/or bicarbonic acid-metal-LFs complex, which is prepared by adding a solution containing LFs to a solution containing a carbonic acid ion and/or a bicarbonic acid ion and a solution containing one kind or multiple kinds of metals and mixing them, with a protein degrading enzyme such as trypsin, pepsine, or chymotrypsin or with an acid or alkali.

As the metals which can be used in the present invention, ferric chloride, copper gluconate, zinc gluconate, manganese (II) chloride, cobalt chloride, nickel chloride aluminum chloride and the like may be used, or sulfates and phosphates of various metals may also be used. Organic compounds such as heme iron may also be used as those metal compounds. The addition amount of each metal is preferably 3 M or more in terms of each metal ion based on 1 M of the LFs. When the amount of one kind of metal added is larger than 1,000 M, precipitation may occur during production or storage, or the metal ion does not bind completely.

Examples of the solution containing carbonic acid and/or bicarbonic acid which can be used in the present invention include carbonic acid solution, an ammonium bicarbonate solution, a sodium bicarbonate solution, a potassium bicarbonate solution, a sodium carbonate solution, a calcium carbonate solution and the like. Those solutions may also be used in mixture. Those solutions may contain other components such as carbohydrate, protein, fat or the like.

### Effect of the Invention

The whitening agent of the present invention exhibits an excellent whitening effect by ingesting the agent transcutaneously or orally. In addition, the ingestion of the whitening agent is safe.

### Best Mode for carrying out the Invention

The whitening agent of the present invention can be used by formulating into a tablet, capsule, granule, suspension, powder, dust, syrup, drink, external preparation or the like in accordance with conventional methods. The whitening agent may be used by mixing with a nutritional supplement, a food and drink such as yoghurt, milk beverage, wafer, bread, snack, cake, pudding, beverage, fermentedmilk, noodle, sausage, various milk powders and baby food, a feed, a cosmetic and a drug. The whitening agent of the present invention may be used alone as a whitening agent and may also be used in combination with a raw material which is generally contained in other foods, feeds, cosmetics, and drugs, such as carbohydrate, lipid, flavor, vitamin, mineral, flavonoid, polyphenol or the like.

The whitening agent of the present invention is desirably ingested in an amount of 2 mg or more per day for each adult in order to obtain its excellent whitening effect. To that end, the whitening agent is desirably contained in an amount of 0.001 to 30% (weight/weight), preferably 0.1 to 10% (weight/weight) in a food, drink, feed, cosmetic, or drug, depending on their forms.

The whitening agent of the present invention can be used in common cosmetics such as emulsion, cream, lotion, face pack and the like. Those cosmetics may be produced by commonly used methods, and the whitening agent of the present invention may be appropriately mixed during the production process of those cosmetics. Further, cosmetics can also be produced from those cosmetics as starting materials. The amount of the whitening agent of the present invention contained in each cosmetic is not particularly limited but generally 0.001 to 30% (weight/weight), preferably 0.1 to 10% (weight/weight) based on the total mass.

The present invention is hereinafter described in more detail by way of examples and test examples, but those examples and test examples are just for exemplification and the present invention is not limited thereby at all.

### Example 1

### (Preparation of bicarbonic acid-metal-LFs complex)

After the following solutions were prepared, they were mixed to prepare a bicarbonic acid-metal-LFs complex.
(Solution A) 1 liter of a solution containing 1 M of sodium bicarbonate
(Solution B1) 0.2 liter of a solution containing each metal salt at each concentration
The following must be noted. In the case of a solution containing one kind of metal, a solution containing 100 mM of a metal salt was prepared. In the case of a solution containing two kinds of metals, equal amounts of metal salts were mixed together to prepare a solution containing 200 mM of metal salts. In the case of a solution containing three kinds of metals, equal amounts of metal salts were mixed together to prepare a solution containing 300 mM of metal salts.
(Solution B2) 0.8 liter of a solution containing 1 mM of LFs

Solution B obtained by mixing Solution B1 and Solution B2 was first added to Solution A, and the resulting mixture was stirred to prepare a solution containing the LFs to which each of the metals had been bound. 300 ml of this solution were dialyzed against ultrapure water by means of a dialysis membrane having a molecular weight of 10 kDa, and desalinated completely to remove unbound metals, after that, the resultant was freeze-dried, and the amounts of metals bound to the LFs were measured by inductively-coupled plasma optical emission spectrometry (ICP). The results are shown in Table 1. As a result, a bicarbonic acid-metal-LFs complex could be obtained at a yield of 90% or more based on the LF. The obtained bicarbonic acid-metal-LFs complex can be used as the whitening agent of the present invention.

**[Table 1]**

| | Metal salt | Concentration (mM) | Metal/LFs (molar ratio) |
|---|---|---|---|
| 1. | Iron | 3 | 3 |
| 2. | | 70 | 70 |
| 3. | | 200 | 200 |
| 4. | Copper | 10 | 4 |
| 5. | | 100 | 32 |
| 6. | | 500 | 160 |
| 7. | Zinc | 50 | 8 |
| 8. | | 100 | 15 |
| 9. | | 500 | 80 |
| 10. | Manganese | 100 | 9 |
| 11. | | 1,000 | 47 |
| 12. | | 2,000 | 178 |
| 13. | Cobalt | 100 | 7 |
| 14. | | 1,000 | 70 |
| 15. | | 2,000 | 142 |
| 16. | Nickel | 100 | 8 |
| 17. | | 1,000 | 80 |
| 18. | | 2,000 | 161 |
| 19. | Aluminum | 300 | 3 |
| 20. | | 1,000 | 10 |
| 21. | | 5,000 | 50 |
| 22. | Iron + copper | 100+100 | 98+62 |
| 23. | Iron + manganese | 100+100 | 92+31 |
| 24. | Iron + nickel | 100+100 | 94+24 |
| 25. | Iron + aluminum | 100+100 | 81+8 |
| 26. | Iron + cobalt | 100+100 | 87+12 |
| 27. | Copper + zinc | 100+100 | 67+31 |
| 28. | Copper + manganese | 100+100 | 62+11 |
| 29. | Iron + copper + zinc | 100+100 | 96+60+29 |

In the table, when two metal salts were used, their molar ratios were given in the order of the metal salts. For example, "98+62" in the column of "iron + copper" means 98 mM of iron and 62 mM of copper. The same applies to the tables below. Note that, as for the metal salts used in this test, ferric chloride was used as an iron salt, copper gluconate was used as a copper salt, zinc gluconate was used as a zinc salt, manganese (II) chloride was used as a manganese salt, cobalt chloride was used as a cobalt salt, nickel chloride was used as a nickel salt, and aluminum chloride was used as an aluminum salt.

### Example 2

### (Preparation of degradation product of bicarbonicacid-metal-LFs complex)

After the following solutions were prepared, they were mixed together to prepare a degradation product of a bicarbonic acid-metal-LFs complex. trypsin (type III, manufactured by Sigma Co., Ltd.) was added to the bicarbonic acid-metal-LFs complex obtained in Example 1 so as to become 1 wt% of trypsin concentration in the complex, and the obtained mixture was hydrolyzed at 37 °C for 24 hours. Thereafter, same amount (1 wt%) of trypsin was further added, and hydrolysis was carried out at 37°C for 24 hours to hydrolyze the bicarbonic acid-metal-LFs complex completely. Note that, for performing this hydrolytic reaction, the reaction solution was sterilized through a sterilization filter to inhibit the growth of bacteria, and then, subjected to the reaction after one drop of toluene was added. Then, the reaction solution was dialyzed by means of a dialysis membrane having a molecular weight of 1 kDa and freeze-dried to obtain a degradation product of the bicarbonic acid-metal-LFs complex having a molecular weight of mainly 55 kDa, 30 kDa, and 10 kDa and including peptides having a molecular weight of 1 kDa or more as another component. The amounts of metals bound to the degradation product of the bicarbonic acid-metal-LFs complex were measured by ICP. The results are shown in Table 2. The obtained degradation product of the bicarbonic acid-metal-LFs complex may be used as the whitening agent of the present invention.

**[Table 2]**

| | Degradation product of bicarbonic acid-metal-LFs complex | Metal/LFs (molar ratio) |
|---|---|---|
| 1. | Iron LF (3) | 3 |
| 2. | Iron LF (70) | 68 |
| 3. | Iron LF (200) | 198 |
| 4. | Copper LF (4) | 3 |
| 5. | Copper LF (32) | 29 |
| 6. | Copper LF (160) | 157 |
| 7. | Iron + copper LF (98+62) | 96+59 |
| 8. | Iron + manganese LF (92+31) | 89+28 |

### Example 3

### (Preparation of bicarbonic acid-metal-LFs degradation product complex)

After the following solutions were prepared, they were mixed to prepare a bicarbonic acid-metal-LFs degradation product complex.
(Solution A) 1 liter of a solution containing 1 M of sodium bicarbonate
(Solution B1) 0.2 liter of a solution containing each metal salt at each concentration
The following must be noted. In the case of a solution containing one kind of metal, a solution containing 100 mM of a metal salt was prepared. In the case of a solution containing two kinds of metals, equal amounts of metal salts were mixed together to prepare a solution containing 200 mM of metal salts. In the case of a solution containing three kinds of metals, equal amounts of metal salts were mixed together to prepare a solution containing 300 mM of metal salts.
(Solution B2) 0.8 liter of a solution prepared by dissolving an LFs degradation product in water so that the concentration of the LFs were 13.2 µM/L in LFs equivalent
Note that, the LFs degradation product which was obtained in accordance with the method of Reference Example 1 in Japanese Patent No. 3223958 was used. Solution B obtained by mixing Solution B1 and Solution B2 was first added to Solution A, and the resulting mixture was stirred to prepare a solution containing the LFs degradation product to which metals were bound. This solution was dialyzed against ultrapure water by means of a dialysis membrane (MWCO [molecular weight cut off]: 1kDa) at 4°C for 90 hours to desalinate completely. After that the retentate was freeze-dried, and the amounts of metals bound to the LFs degradation product were measured by ICP. The results are shown in Table 3. The obtained bicarbonic acid-metal-LFs degradation product may be used as the whitening agent of the present invention.

**[Table 3]**

| | Metal salt | Concentration (mM) | Metal/LFs (molar ratio) |
|---|---|---|---|
| 1. | Iron | 3 | 3 |
| 2. | | 70 | 70 |
| 3. | | 200 | 201 |
| 4. | Copper | 10 | 4 |
| 5. | | 100 | 33 |
| 6. | | 500 | 162 |
| 7. | Iron + copper | 100+100 | 93+75 |
| 8. | Copper + zinc | 100+100 | 66+35 |
| 9. | Iron + copper + zinc | 100+100+100 | 94+55+27 |

Note that, as for the metal salts used in this test, ferric chloride was used as an iron salt, copper gluconate was used as a copper salt, zinc gluconate was used as a zinc salt, manganese (II) chloride was used as a manganese salt, cobalt chloride was used as a cobalt salt, nickel chloride was used as a nickel salt, and aluminum chloride was used as an aluminum salt.

### [Test Example 1]

### (Assay of melanin formation inhibiting effect)

The malignant melanoma B16-F0 cell of a mouse (Dainippon Sumitomo Pharmaceutical Co., Ltd.) was used in the experiment. An Eagle's MEM medium containing 10% of a bovine fetal serum (Sigama-Aldrich Co., Ltd.) was used and the cell was cultured in a CO₂ incubator (5% CO₂, 37°C). A suspension having a cell concentration of 3×10⁵ B16 cells/ml was prepared, and 1 ml of this suspension was each dispensed a into 100 mm dish containing 9 ml of a medium. On the following day, the media were replaced by another media containing 0.01 to 1% of LF, the bicarbonic acid-metal-LF complex (FeLF (70), FeLF (200), CuLF (32), CuLF (160), Fe + CuLF (98+62)) obtained in Example 1, and a metal salt and an LF or an LF degradation product, and the cells were cultured for 4 days. After the completion of the culture, the cells were taken up, the number of cells of each group was set to 5×10⁶, and subjected to centrifugation. 500 ml of 1 M NaOH were added and dissolved, and the absorbance at 405 nm of the resulting solution was measured by a spectrophotometer. The melanin formation inhibition ratio of each group was calculated assuming that the inhibition ratio of a group of cells obtained without adding 1 M NaOH was defined as 100%, and the results are shown in Table 4 below.

**[Table 4]**

| Sample | Concentration (mass%) | Inhibition ratio (%) |
|---|---|---|
| LF | 0.01 | 12.3 |
| | 0.1 | 16.1 |
| | 1 | 18.4 |
| FeLF (70) (Example 1) | 0.01 | 21.3 |
| | 0.1 | 29.4 |
| | 1 | 40.1 |
| FeLF (200) (Example 1) | 0.01 | 21.1 |
| | 0.1 | 31.8 |
| | 1 | 38.0 |
| LF + ferric chloride (70) | 0.01 | 14.0 |
| | 0.1 | 17.9 |
| | 1 | 21.3 |
| LF + ferric chloride (200) | 0.01 | 13.8 |
| | 0.1 | 18.7 |
| | 1 | 21.8 |
| CuLF (32) (Example 1) | 0.01 | 15.2 |
| | 0.1 | 21.5 |
| | 1 | 32.9 |
| CuLF (160) (Example 1) | 0.01 | 16.5 |
| | 0.1 | 22.0 |
| | 1 | 36.1 |
| LF + copper (II) chloride (32) | 0.01 | 13.0 |
| | 0.1 | 18.3 |
| | 1 | 20.0 |
| LF + copper (II) chloride (160) | 0.01 | 12.9 |
| | 0.1 | 19.0 |
| | 1 | 20.6 |
| Fe + CuLF (98+62) | 0.01 | 18.8 |
| | 0.1 | 24.6 |
| | 1 | 39.0 |
| LF + ferric chloride (98) + copper(II) chloride (62) | 0.01 | 13.7 |
| | 0.1 | 19.0 |
| | 1 | 21.6 |

As a result, the melanin formation inhibition effect of the LF was observed at concentration of 0.01% to 1% in a concentration dependent manner. On the other hand, almost no increase in melanin formation inhibition effect was observed in a solution prepared by adding an LF, ferric chloride, and copper (II) chloride at the same time as compared with the solution adding LF alone. However, in the case of FeLF (70), FeLF (200), CuLF (32), CuLF (160) and Fe + CuLF (98+62), the formation of melanin was inhibited at a concentration of 0.01% to 1% in a concentration dependent manner, and it was recognized that their melanin formation inhibiting effects were stronger than that of the LF alone. This shows that the whitening agent of the present invention has a higher whitening effect than the case where the LF, ferric chloride, and copper(II) chloride are used alone or at the same time.

### [Test Example 2]

### (Assay of tyrosinase activity inhibiting effect)

Tyrosinase is an enzyme which is involved in a pathway for synthesizing melanin from tyrosine and converts tyrosine into dopa and then dopa into dopaquinone. Then, to examine the inhibition effect for a melanin synthesis pathway of the whitening agent of the present invention, a tyrosinase activity inhibiting effect was tested.

Tyrosinase (derived from mushroom; Sigama-Aldrich Corp.) was used, and an LF, the bicarbonic acid-metal-LFs complex obtained in Example 1, the degradation product of the carbonic acid-metal-LFs complex obtained Example 2, the bicarbonic acid-metal-LFs degradation product complex obtained in Example 3, or a metal salt was added to carry out a pretreatment at 37°C for 15 minutes in the experiment. Dopa (Sigama-Aldrich Co., Ltd.) was added as a substrate and the mixture was further reacted at 37 °C for 5 minutes, after that the reaction product was measured the absorbance at 476 nm. The inhibition ratio was calculated assuming that the inhibition ratio of a product obtained without any addition was defined as 100%. The results obtained when the bicarbonic acid-metal-LFs complex was used are shown in Table 5, the results obtained when the degradation product of the bicarbonic acid-metal-LFs complex was used are shown in Table 6, and the results obtained when the bicarbonic acid-metal-LFs degradation product complex was used are shown in Table 7.

**[Table 5]**

| Bicarbonic acid-metal-LF complex | | | |
|---|---|---|---|
| | Inhibition ratio | | |
| Sample (molar ratio) | 0.001% | 0.01% | 0.1% |
| LF | 10.5% | 11.1% | 12.4% |
| Iron LF (3) | 11.3% | 15.9% | 20.0% |
| Iron LF (70) | 25.3% | 30.4% | 35.2% |
| Iron LF (200) | 27.3% | 29.1% | 30.4% |
| Ferric chloride | 4.9% | 5.5% | 10.1% |
| Copper LF (4) | 11.5% | 12.0% | 15.0% |
| Copper LF (32) | 18.0% | 20.8% | 26.1% |
| Copper LF (160) | 22.3% | 28.4% | 30.1% |
| Copper(II) chloride | 0.5% | 0.3% | 0.3% |
| Zinc LF (8) | 12.1% | 16.1% | 19.2% |
| Zinc LF (15) | 15.8% | 19.5% | 20.4% |
| Zinc LF (80) | 17.9% | 19.2% | 18.0% |
| Zinc(II) chloride | 2.3% | 3.0% | 6.1% |
| Manganese LF (9) | 13.7% | 17.2% | 18.9% |
| Manganese LF (47) | 14.5% | 18.0% | 20.5% |
| Manganese LF (178) | 20.1% | 23.5% | 27.0% |
| Manganese(II) chloride | 3.8% | 3.9% | 5.1% |
| Cobalt LF (7) | 16.2% | 16.8% | 16.6% |
| Cobalt LF (70) | 19.0% | 18.7% | 20.3% |
| Cobalt LF (142) | 21.0% | 26.7% | 28.6% |
| Cobalt chloride | 5.2% | 5.8% | 7.9% |
| Nickel LF (8) | 18.0% | 20.0% | 21.8% |
| Nickel LF (80) | 22.6% | 25.9% | 28.8% |
| Nickel LF (161) | 20.4% | 23.8% | 25.9% |
| Nickel chloride | 4.3% | 5.9% | 7.5% |
| Aluminum LF (3) | 14.2% | 18.7% | 19.0% |
| Aluminum LF (10) | 18.1% | 19.6% | 20.0% |
| Aluminum LF (50) | 22.1% | 25.8% | 28.1% |
| Aluminum chloride | 0.7% | 1.1% | 2.9% |
| Iron + copper LF (98+62) | 23.3% | 25.6% | 30.1% |
| Iron + manganese LF (92+31) | 20.7% | 24.6% | 28.0% |
| Iron + nickel LF (94+24) | 20.6% | 23.8% | 26.7% |
| Iron + aluminum LF (81+8) | 22.7% | 24.9% | 29.6% |
| Iron + cobalt LF (87+12) | 21.0% | 23.7% | 27.5% |
| Copper + zinc LF (67+31) | 19.5% | 23.8% | 25.9% |
| Copper + manganese LF (62+11) | 19.1% | 22.1% | 25.8% |
| Iron+copper + zincLF (96+60+29) | 23.7% | 25.4% | 29.9% |
| LF + ferric chloride (3) | 10.8% | 12.5% | 13.8% |
| LF + ferric chloride (70) | 11.7% | 14.3% | 16.1% |
| LF + ferric chloride (200) | 11.3% | 13.9% | 17.2% |
| LF + copper(II) chloride (4) | 10.8% | 11.9% | 12.4% |
| LF + copper (II) chloride (30) | 11.2% | 13.7% | 13.9% |
| LF + copper(II) chloride (160) | 11.1% | 13.5% | 14.3% |

**[Table 6]**

| Degradation product of bicarbonic acid-metal-LF complex | | | |
|---|---|---|---|
| | Inhibition ratio | | |
| Sample (molar ratio) | 0.001% | 0.01% | 0.1% |
| Degradation product of LF | 10.2% | 12.0% | 13.1% |
| Degradation product of iron LF (3) complex | 10.6% | 14.9% | 19.2% |
| Degradation product of iron LF (70) complex | 23.3% | 28.2% | 33.1% |
| Degradation product of iron LF (200) complex | 25.1% | 27.8% | 28.0% |
| Degradation product of copper LF (4) complex | 10.6% | 12.2% | 14.2% |
| Degradation product of copper LF (32) complex | 17.2% | 18.9% | 24.1% |
| Degradation product of copper LF (160) complex | 21.5% | 26.7% | 28.6% |
| Degradation product of iron + copper LF (98+62) complex | 21.5% | 23.8% | 28.6% |
| Degradation product of iron + manganese LF (92+31) complex | 19.3% | 23.3% | 26.1% |

**[Table 7]**

| Complex of bicarbonic acid-metal-LF degradation product | | | |
|---|---|---|---|
| | Inhibition ratio | | |
| Sample (molar ratio) | 0.001% | 0.01% | 0.1% |
| LF | 10.5% | 11.1% | 12.4% |
| LF degradation product | 10.2% | 12.0% | 13.1% |
| Iron LF degradation product (3) | 12.6% | 16.6% | 21.5% |
| Iron LF degradation product (70) | 25.9% | 29.7% | 33.9% |
| Iron LF degradation product (201) | 27.8% | 30.1% | 31.2% |
| Copper LF degradation product (4) | 12.1% | 13.8% | 16.2% |
| Copper LF degradation product (33) | 17.8% | 21.3% | 26.9% |
| Copper LF degradation product (162) | 23.0% | 28.2% | 29.3% |
| Iron + copper LF degradation product (93+75) | 22.3% | 26.2% | 28.2% |
| Copper + zinc LF degradation product (66+35) | 20.5% | 24.6% | 27.2% |
| Iron + copper + zinc LF degradation product (94+55+27) | 22.9% | 24.8% | 29.0% |
| LF degradation product + ferric chloride (3) | 10.8% | 12.3% | 13.5% |
| LF degradation product + ferric chloride (70) | 11.2% | 12.5% | 13.9% |
| LF degradation product + ferric chloride (200) | 11.9% | 13.0% | 13.2% |
| LF degradation product + copper(II) chloride (4) | 10.5% | 11.9% | 12.3% |
| LF degradation product + copper(II) chloride (30) | 10.5% | 12.0% | 13.1% |
| LF degradation product + copper(II) chloride (160) | 11.0% | 12.8% | 13.9% |

The above results demonstrate that LF significantly inhibited the activity of the enzyme in the melanin synthesis pathway at concentration of 0.01% to 1% in a concentration dependent manner. It was found that the bicarbonic acid-metal-LF complex inhibited the activity of the enzyme at a concentration of 0.001% to 0. 1% in a concentration dependent manner and that the inhibition was increased by forming a complex regardless of the type and number of metals. The degradation product of the bicarbonic acid-metal-LF complex and the bicarbonic acid-metal-LF degradation product complex also exhibited similar effect. The inhibition effects of the bicarbonic acid-metal-LF complex, the degradation product thereof, and the bicarbonic acid-metal-LF degradation product complex are stronger than that of an LF alone or an degradation product thereof alone, or a co-existing composition of an LF and a metal or an LF degradation product and a metal. It was found that a higher whitening effect was obtained by using as a complex.

### [Test Example 3]

### (Assay of melanin degradation/excretion promoting effect by oral administration)

Hair was removed from the back of an A-1-line female guinea pig having a weight of about 400 g, and ultraviolet radiation (30.3 kj/m2 of UVA (max. 360 nm), 4.8 kJ/m2 of UVB (max. 312 nm)) was irradiated to the back once a day for 4 days. Thereafter, the guinea pigs were divided into the following four test groups (each group had 10 guinea pigs). Physiological saline was administered to guinea pigs in an amount of 5 ml per kg weight (group A), FeLF (70) (Example 1) was administered to guinea pigs in an amount of 2 mg/5 ml per kg weight (group B), FeLF (70) was administered to guinea pigs in a amount of 5 mg/5 ml per kg weight (group C), and FeLF (70) was administered to guinea pigs in an amount of 10 mg/5 ml per kg weight (group D). Those four groups were administered orally using feeding needle once a day and bred for 4 weeks. The influence upon the pigmentation to the back skins of the guinea pigs was measured by a colorimeter (Chroma Meter CR-200; Minolta Corporation) at the start and end of the administration period. The recovery ratio was calculated from the difference of brightness between before and after exposure of ultraviolet radiation assuming that the brightness before exposure was defined as 100%. The results are shown in Table 8.

**[Table 8]**

| Group | FeLF (70) dosage (mg/kg) | Brightness recovery ratio (%) |
|---|---|---|
| A group | 0 | 20.0 |
| B group | 2 | 31.8 |
| C group | 5 | 43.9 |
| D group | 10 | 63.2 |

Table 8 shows that FeLF (70) exhibited a remarkable brightness improving effect as compared with that of a control group and the effect was dependent upon concentration. It was made clear from the results that the whitening agent of the present invention showed the effect of preventing and improving pigmentation by oral administration remarkably.

### [Test Example 4]

### (Assay of melanin degradation/excretion promoting effect by application)

Hair was removed from the back of an A-1-line female guinea pig having a weight of about 400 g and UV (30.3 kJ/m2 of UVA (max. 360 nm), 4.8 kJ/m2 of UVB (max. 312 nm)) was irradiated to the back once a day for 4 days. Thereafter, the sample was continuously applied to the site to be tested twice a day for 4 weeks. The sample to be tested was dissolved in a mixture of water, ethanol, and propylene glycol in a ratio of 2:2:1 and applied. The guinea pigs were divided into four test groups (each group had 10 guinea pigs) : a control group (group A), 0.01% FeLF (70) (Example 1) (group B), 0.1% FeLF (70) (group C), and 1% FeLF (70) (group D). The influence upon the pigmentation to the back skins of the guinea pigs was measured by a colorimeter (Chroma Meter CR-200; Minolta Corporation) at the start and end of the administration period. The recovery rate was calculated from the difference of brightness between before and after exposure of ultraviolet radiation assuming that the brightness before exposure was defined as 100%. The recovery ratio was calculated from the difference of brightness between before and after exposure of ultraviolet radiation assuming that the brightness before exposure was defined as 100%. The results are shown in Table 9.

**[Table 9]**

| Group | FeLF (70) Concentration (mg/kg) | Brightness recovery ratio (%) |
|---|---|---|
| A group | 0 | 19.9 |
| B group | 0.01 | 38.1 |
| C group | 0.1 | 64.3 |
| D group | 1 | 79.6 |

Table 9 shows that FeLF (70) was observed the melanin degradation/excretion promotion effect at a concentration range of 0.01 to 1% in a concentration dependent manner as compared with the control group. This result shows that the whitening agent of the present invention has a remarkable pigmentation preventing and improving effect by directly applying to the skin.

It was made clear from the above results that the whitening agent of the present invention had the actions of inhibiting the melanin synthesis pathway and promoting the degradation or excretion of melanin. It was also made clear that a whitening effect is obtained without regard to transcutaneous administration or oral administration. The whitening agent of the present invention may be thought to exhibit a higher whitening effect because it acts on plural systems in which melanin is involved.

### Example 4

### (Production of whitening cream)

Whitening cream was produced using the whitening agent of Example 1 by mixing the materials in a ratio shown in Table 10.

**[Table 10]**

| | |
|---|---|
| Glycerin monostearate (self-emulsifying type) | 10.0 |
| Purified lanolin | 6.0 |
| Fluid paraffin | 5.0 |
| Jojoba oil | 5.0 |
| Paraben | 0.3 |
| FeLF (200) (Example 1) | 0.3 |
| Fragrance | Appropriate amount |
| Sterilized ion exchanged water | Total amount 100.0 |

### Example 5

### (Production of whitening lotion)

Whitening lotion was produced using the whitening agent of Example 1 by mixing the materials in a ratio shown in Table 11.

**[Table 11]**

| | |
|---|---|
| Sorbitol | 3.0 |
| DL-sodium pyrrolidone carboxylate | 2.0 |
| Carboxymethyl cellulose | 0.3 |
| Paraben | 0.1 |
| ZnLF (15) (Example 1) | 0.1 |
| Fragrance | Appropriate amount |
| Sterilized ion exchanged water | Total amount 100.0 |

### Example 6

### (Production of whitening tablet)

The whitening tablet of the present invention was produced using the whitening agent of Example 1 by mixing raw materials in a ratio shown in Table 12 and forming and tableting the resulting mixture into a 1 g tablet in accordance with a conventional method.

**[Table 12]**

| | |
|---|---|
| Hydrated crystal of glucose | 83.5 (wt%) |
| FeLF (70) (Example 1) | 10.0 |
| Mineral mixture | 5.0 |
| Sugar ester | 1.0 |
| Fragrance | 0.5 |

Note that, 100 mg of FeLF (70) were contained in 1 g of this whitening tablet.

### Example 7

### (Preparation of whitening liquid nutrition composition)

A whitening liquid nutrition composition was prepared using the whitening agent of Example 1 in accordance with the following method. 50 g of CuLF (32) of Example 1 and 1,000 g of casein were dissolved in 3, 950 g of deionized water, and the resulting solution was heated up to 50°C, and mixed and stirred by means of a TK homomixer (TK ROBO MICS; manufactured by Tokushu Kika Kogyo Co., Ltd.) at 6,000 rpm for 30 minutes to obtain a CuLF solution having a CuLF content of 50 g/5 kg. 4.0 kg of casein, 5.0 kg of soya protein, 1.0 kg of fish oil, 3.0 kg of perilla oil, 18.0 kg of dextrin, 6.0 kg of a mineral mixture, 1.95 kg of a vitaminmixture, 2.0 kg of an emulsifier, 4.0 kg of a stabilizer, and 0.05 kg of a fragrance were mixed with 5.0 kg of the CuLF solution, and the resulting mixture was charged into a 200 ml retort pouch and sterilized by a retort sterilization apparatus (class 1 pressure vessel, TYPE: RCS-4CRTGN, manufactured by Hisaka Works, Ltd.) at 121°C for 20 minutes to produce 50 kg of the whitening liquid nutrition composition of the present invention. Note that, this whitening liquid nutrition composition contained CuLF (32) in an amount of 100 mg for each 100 g.

### Example 8

### (Preparation of whitening gelatinous food)

A whitening gelatinous food was prepared using the whitening agent of Example 2 in accordance with the following method. 2 g of the CuLF (32) degradation product of Example 2, 10 g of pectin, and 10 g of a whey protein concentrate were dissolved in 978 g of deionized water, and the resulting solution was mixed and stirred by an ultradisperser (ULTRA-TURRAX T-25; IKA Japan Co., Ltd.) at 9, 500 rpm for 3 minutes. Thereafter, 80 g of sorbitol, 4 g of an acidulant, 4 g of a fragrance, 2 g of calcium lactate, and 910 g of water were added to the above solution, and the resulting solution was mixed and stirred to prepare the whitening gelatinous food of the present invention. This food was charged into a 200 ml cheer pack and sterilized at 85°C for 20 minutes, and the pack was sealed tightly to prepare 10 packs of the whitening gelatinous food of the present invention. Note that, this whitening gelatinous food contained the CuLF (32) degradation product in an amount of 100 mg for each 100 g.

### Example 9

### (Preparation of whitening drink)

A whitening drink was prepared using the whitening agent of Example 3 in accordance with the following method. After 300 g of skimmed milk powder was dissolved in 409 g of deionized water, 1 g of the FeLF degradation product (201) of Example 3 was dissolved in the resulting solution and heated up to 50°C, and the resulting solution was mixed and stirred by an ultradisperser (ULTRA-TURRAX T-25; manufactured by IKA Japan Co., Ltd.) at 9,500 rpm for 30 minutes. 100 g of maltitol, 2 g of an acidulant, 20 g of reduced starch syrup, 2 g of a fragrance, and 166 g of deionized water were added to the solution, the resulting solution was charged into a 100 ml glass bottle and sterilized at 90°C for 15 minutes, and the bottle was sealed tightly. Thus, 10 bottles (each containing 100 ml) of the whitening drink of the present invention were prepared. Note that this whitening drink contained the FeLF degradation product (201) in an amount of 100 mg for each 100 ml.

### Example 10

### (Preparation of whitening feed for dogs)

A whitening feed for dogs was prepared using the whitening agent of Example 1 in accordance with the following method. 0.2 kg of FeLF (70) of Example 1 was dissolved in 99.8 kg of deionized water, and the resulting solution was heated up to 50°C, then mixed and stirred by a TK homomixer (MARK II TYPE 160; manufactured by Tokushu Kika Kogyo Ltd.) at 3, 600 rpm for 40 minutes to obtain a FeLF solution having a FeLF content of 2 g/100 g. 12 kg of soymeal, 14 kg of skimmed milk powder, 4 kg of soybean oil, 2 kg of corn oil, 23.2 kg of palm oil, 14 kg of cornstarch, 9 kg of flour, 2 kg of bran, 5 kg of a vitamin mixture, 2.8 kg of cellulose, and 2 kg of a mineral mixture were mixed with 10 kg of the FeLF solution, and the resulting mixture was sterilized at 120°C for 4 minutes to produce 100 kg of the whitening feed for dogs of the present invention. Note that, this whitening feed for dogs contained FeLF (70) in an amount of 20 mg for each 100 g.

### Industrial Applicability

The whitening agent of the present invention including a carbonic acid and/or bicarbonic acid-metal-LFs complex, a degradation product of the complex, and a carbonic acid and/or bicarbonic acid-metal-LFs degradation product complex as active ingredients has an excellent skin whitening effect and is useful for the prevention and treatment of spots, freckles, and the like. In addition, the product of the present invention has high safety and an excellent whitening effect on the skin. The present invention can be further used as a whitening food, drink, feed, cosmetic, and drug including this whitening agent.

## Claims

1. A complex of lactoferrins or a degradation product thereof, a metal, and carbonic acid and/or bicarbonic acid for use as an active ingredient of a whitening agent.

2. The complex for use according to claim 1, wherein the complex is obtained by mixing a solution containing a carbonic acid ion and/or a bicarbonic acid ion, a solution containing one kind or multiple kinds of metals, and a solution containing lactoferrins or a degradation product thereof.

3. The complex for use according to claim 1 which is a degradation product obtained by hydrolyzing a complex of lactoferrins, a metal, and carbonic acid and/or bicarbonic acid for use as an active ingredient of a whitening agent.

4. The complex for use according to any one of claims 1 to 3, wherein the complex contains 3 to 1,000 molecules of one kind or multiple kinds of metals and 15 or more molecules of carbonic acid and/or bicarbonic acid per 1 molecule of lactoferrins or a degradation product thereof.

5. The complex for use according to any one of claims 1 to 4, wherein the metals comprise one kind or two or more kinds of metals selected from iron, copper, zinc, manganese, cobalt, nickel, and aluminum.

6. The complex for use according to any one of claims 1 to 5 for use as component of a food, drink, feed, cosmetic, or drug.

## Patentansprüche

1. Komplex aus Lactoferrinen oder einem Abbauprodukt davon, einem Metall und Kohlensäure und/oder Hydrogenkohlensäure (bicarbonic acid) zur Verwendung als ein Wirkstoff von einem Bleichmittel.

2. Komplex zur Verwendung nach Anspruch 1, wobei der Komplex erhalten wird, indem eine Lösung, die ein Kohlensäureion (carbonic acid ion) und/oder ein Hydrogenkohlensäureion (bicarbonic acid ion) enthält, eine Lösung, die eine Art oder mehrere Arten von Metallen enthält, und eine Lösung, die Lactoferrine oder ein Abbauprodukt davon enthält, gemischt werden.

3. Komplex zur Verwendung nach Anspruch 1, der ein Abbauprodukt ist, das erhalten wird, indem ein Komplex aus Lactoferrinen, einem Metall und Kohlensäure und/oder Hydrogenkohlensäure (bicarbonic acid) hydrolisiert wird, zur Verwendung als ein Wirkstoff von einem Bleichmittel

4. Komplex zur Verwendung nach einem der Ansprüche 1 bis 3, wobei der Komplex 3 bis 1000 Moleküle von einer Art oder von mehreren Arten von Metallen und 15 oder mehr Moleküle Kohlensäure und/oder Hydrogenkohlensäure (bicarbonic acid) pro 1 Molekül der Lactoferrine oder eines Abbauproduktes davon enthält.

5. Komplex zur Verwendung nach einem der Ansprüche 1 bis 4, wobei die Metalle eine Art oder zwei Arten oder mehrere Arten von Metallen umfassen, die aus Eisen, Kupfer, Zink, Mangan, Kobalt, Nickel und Aluminium ausgewählt werden.

6. Komplex zur Verwendung nach einem der Ansprüche 1 bis 5, zur Verwendung als eine Komponente von einem Nahrungsmittel, Getränk, Futtermittel, Kosmetikprodukt oder Arzneimittel.

## Revendications

1. Complexe de lactoferrines ou d'un produit de dégradation de celles-ci, de métal, et d'acide carbonique et/ou d'acide bicarbonique à utiliser en tant qu'ingrédient actif d'un agent de blanchiment.

2. Complexe à utiliser selon la revendication 1, dans lequel le complexe est obtenu en mélangeant une solution contenant un ion d'acide carbonique et/ou un ion d'acide bicarbonique, une solution contenant un ou plusieurs types de métaux, et une solution contenant des lactoferrines ou un produit de dégradation de celles-ci.

3. Complexe à utiliser selon la revendication 1 qui est un produit de dégradation obtenu par hydrolyse d'un complexe de lactoferrines, d'un métal, et d' acide carbonique et/ou d' acide bicarbonique à utiliser en tant qu'ingrédient actif d'un agent de blanchiment.

4. Complexe à utiliser selon l'une quelconque des revendications 1 à 3, dans lequel le complexe contient 3 à 1000 molécules d'un ou de plusieurs types de métaux et au moins 15 molécules d'acide carbonique et/ou d'acide bicarbonique par molécule de lactoferrines ou d'un produit de dégradation de celles-ci.

5. Complexe à utiliser selon l'une quelconque des revendications 1 à 4, dans lequel les métaux comprennent un ou deux ou plusieurs types de métaux choisis parmi le fer, le cuivre, le zinc, le manganèse, le cobalt, le nickel et l'aluminium.

6. Complexe à utiliser selon l'une quelconque des revendications 1 à 5 à utiliser en tant que composant d'un aliment, d'un boisson, d'une nourriture, d'un produit cosmétique, ou d'un médicament.
